# EUROPEAN PATENT SPECIFICATION

(11) **EP 1 862 230 B1**
(45) Date of publication and mention of the grant of the patent: **08.01.2014**
(21) Application number: 07109244.9
(22) Date of filing: 30.05.2007
(51) Int. Cl.: A61L 2/22, B08B 9/32, B08B 9/34, B29C 49/36, B29C 49/58, B29C 49/78, B29C 49/42

(54) **An apparatus for washing/sterilising/blowing containers**
Vorrichtung zum Waschen / Sterilisieren / Ausblasen von Behältern
Dispositif de lavage / stérilisation / soufflage de conteneurs

(30) Priority: 31.05.2006 IT PR20060051
(43) Date of publication of application: 05.12.2007
(73) Proprietor: PROMEC S.R.L., 43010 Fontevivo (Parma) (IT)
(72) Inventor: ROCCHI, Flavio, 43012, FONTANELLATO (PARMA) (IT); PIRONDI, Alessandro, 43100, PARMA (IT); VETTORI, Matteo, 43100, PARMA (IT)
(74) Representative: Karaghiosoff, Giorgio Alessandro

(56) References cited:
- WO-A1-2004/045784
- DE-A1- 4 207 346
- DE-B- 1 105 746
- US-A- 3 097 672

## Description

The present invention relates to an apparatus for washing / sterilising / blowing containers, comprising:
- a frame;
- a carrousel, rotating relative to said frame;
- a plurality of nozzles associated with the carrousel, fed with a washing fluid and co-operating with means for gripping the containers to inject the fluid into the containers;
- a plurality of valves associated with the carrousel and operatively connected to corresponding nozzles to enable them / disable them, each valve being provided with a control element movable between a first and a second operative position, corresponding to stable open / closed positions of the valve;
- means for activating / deactivating the valves.

Within the food industry, and in particular in the sector of bottling, i.e. filling containers, use is made of apparatuses dedicated to cleaning and/or conditioning (and possibly sterilising) containers to be filled.

It should be noted that, with regard to the treatment of containers, i.e. cleaning and/or conditioning containers, use of both liquid and gaseous fluids is known. If gaseous fluids are used to condition the containers, said apparatuses are also called "blowers" and the washing fluid is properly defined blowing fluid.

Said apparatuses generally comprise a carrousel, rotating relative to a frame. Operatively, the carrousel receives from a feeding star the containers to be treated and transfers them to an output star, at the end of the treatment, in a continuous process.

The carrousel comprises a plurality of nozzles, in order to enable a plurality of containers to be treated simultaneously. To each nozzle corresponds a pincer, able to withdraw a container from the feeding star, actuate it to prepare it for washing and place it to be fed to the output star.

The actuation of the containers substantially consists of a 180 degree overturn, so that, in said overturned position, the opening of each container is aligned to a nozzle, so the nozzle itself can inject the washing or blowing fluid into the container.

After the washing operation, each container is maintained in the overturned position, in a dripping step; subsequently, the pincers again overturn the containers, to then feed them to the output star. It should be noted that, if the treatment fluid is gaseous, said dripping step is omitted.

Since the nozzles and the pincers are integral with the carrousel and rotate therewith, the steps of withdrawing, washing, dripping (if provided) and transferring the containers to the output correspond to respective angles of rotation of the carrousel. In particular, for each nozzle, with regard to the washing of a container, i.e. the step during which the nozzle injects the washing fluid into the container, the corresponding wash angle (also called flux angle) is given by the position of the nozzle (rotating integrally with the carrousel) relative to the frame between the initial and final instants of the injection of the fluid. Therefore, the duration of the washing step is proportional to the wash angle and inversely proportional to the velocity of rotation of the carrousel relative to the frame. Similarly, the duration of each of said steps is proportional to the angle corresponding to that step and inversely proportional to the velocity of rotation of the carrousel relative to the frame.

With regard to the need to enable / disable the nozzles, a plurality of valves associated with the carrousel is generally used. Each valve is connected to a corresponding nozzle; consequently, an activation / deactivation of a valve entails the enabling / disabling of the corresponding nozzle.

With regard to the manner of activation / deactivation of the vales, several solutions are known.

Some solutions provide for activating / deactivating the valves mechanically, e.g. using cams. While having the merit of being economical, such solutions are disadvantageous, because they require the use of members subject to wear (by rubbing), with the consequent reduction in the reliability of the apparatus; moreover, said solutions require the presence of lubricating means, which present problems also in terms of hygiene.

Other solutions allow, in effect, to avoid the presence of mechanical members. In particular, in the prior art (as described in the patent application PR2004C000048) use is known of a permanent magnet, fastened to the frame at the washing angle, said magnet being able to interact with a valve, when the valve transits above the magnet itself. The action of the magnet is to actuate the valve from a closed position to an open position, the valve being monostable and in particular of the normally closed type. In this way, when the valve, in its own rotation integral with the carrousel, is in position of non interference relative to the magnet, the action of the magnet itself ceases and the valve closes.

However, said solution presents some drawbacks.

First of all, it requires a magnet of considerable dimensions, because it has to cover the entire wash angle, with consequent increase in the costs and in the magnetic forces at play. Moreover, said solution does not allow to change the wash angle. It should be noted that the possibility of changing the wash angle is important for two reasons. The first reason is the need to vary the washing time according to the dimensions of the treated containers. The second reason is given by the need to maintain constant the washing time (for a certain type of treated containers) as the velocity of rotation of the carrousel changes (said velocity of rotation depends on the hourly rate requirements in the container treatment process), to optimise conditioning fluid consumption and reduce the operating costs of the plant.

Other solutions (e.g., the one described in the patent application PR2002A000066) provide for the use of solenoid valves, electrically actuated by receivers, associated with the carrousel, interacting with corresponding emitters, associated with the frame. While such solutions provide for a non continuous adjustment of the washing angle, they are complicated and costly, because of the use of solenoid valves and of electrical / electronic circuits for the control of the valves themselves.

Moreover, the use of electrical devices in the presence of free liquids or of damp environments is hazardous, because it can cause short circuits.

An additional disadvantage is given by the presence of emitters and receivers, which considerably complicate the construction of the machine and its control during operation.

A further disadvantage is given by the fact that said emitters and receivers are subject to malfunctions caused by possible dirt deposits.

An object of the present invention is to eliminate the aforesaid drawbacks and to make available an apparatus for washing / sterilising / blowing containers that is able to command the opening / closing of the valves without mechanical parts in rubbing contact.

Another object of the present invention is to make available an apparatus for washing / sterilising / washing containers that enables to regulate the wash angle, i.e. the time during which the containers are subjected to washing, in continuous or discrete fashion, without stopping the apparatus.

Yet a further object of the present invention is to make available an apparatus for washing / sterilising / blowing containers that is simple, economical and reliable.

The document DE 42 07 346 A1 discloses an apparatus according to the preamble of claim 1. In this apparatus, the magnet of the control element is attracted by the material of the valve housing and, thereby, held in an open position.

The objects indicated above are achieved by the apparatus of the present invention as it is defined in claim 1.

These and other features shall become more readily apparent from the description that follows of a preferred embodiment, illustrated purely by way of non limiting example in the accompanying drawing tables, in which:
- figure 1 schematically shows a partially open top view of an apparatus for washing containers according to the present invention;
- figure 2 schematically shows a partially open top view of the apparatus of figure 1 according to an embodiment variant;
- figure 3 schematically shows a partially open top view of the apparatus of figure 1 according to an additional embodiment variant;
- figure 4 shows a partially sectioned top view of a detail of the apparatus of figure 3;
- figure 5 shows a partially sectioned top view of the detail of figure 3, according to an embodiment variant;
- figure 6 shows a sectioned view of a valve of the apparatus according to the present invention, in inactive stable position;
- figure 7 shows a sectioned view of the valve of figure 6 interacting with an opening magnet;
- figure 8 shows a sectioned view of the valve of figure 6 interacting with an opening magnet;
- figure 9 shows a sectioned view of the valve of figure 6 interacting with a closing magnet;
- figure 10 shows the detail of figure 3, according to an additional embodiment variant.

In the figures, the reference number 1 designates an apparatus for washing / sterilising / blowing containers 2, according to the present invention. It should be noted that what is described and illustrated below also applies for apparatuses for blowing containers, i.e. conditioning apparatuses that use gaseous fluids for washing containers. The characteristics and the technical problems described for washing or sterilising containers are the same, in the case of blowing the containers. Preferably, the blowing apparatuses are used in the cases in which no liquid residue in the container is tolerated, e.g. when bottling liquor or oil for human consumption.

With regard to the treatment fluid, it will indifferently be constituted:
- by a cleaning / sterilising fluid (in particular a liquid);
- by a gaseous fluid (for blowing), in particular air, steam, sterilising or inert gases.

The apparatus 1 comprises a frame 3, a carrousel 4 rotating relative to said frame 3, a plurality of nozzles (not shown because they are known in themselves) associated with the carrousel 4, fed with a washing or blowing fluid and co-operating with means (also not shown because they are known in themselves) for gripping the containers 2 to inject the fluid into the containers, washing them. The gripping means can be constituted for example by pincers.

The apparatus 1 operates continuously; in particular, the gripping means withdraw the containers 2 to be washed from a feeding star 5, moving each container 2 in such a way as to align it to a corresponding nozzle (in general, they overturn it); subsequently, the containers 2 are kept overturned for a certain distance, to enable their dripping; lastly, the gripping means transfer each container 2 to an output star 6, overturning the containers 2 again.

It should be noted that the nozzles and the gripping means are integral with the carrousel 4 and rotate therewith; therefore, there are defined steps of withdrawing, washing, dripping and transferring the containers 2 to the output, which correspond to respective angles of rotation of the carrousel: angle of withdrawal / overturn α, wash angle β, drip angle γ and transfer / overturn angle δ , respectively.

It should be noted that, in the case of a blowing apparatus, the dripping step is not generally included, whilst the other steps are provided. Alternatively, the drip step is replaced by a short dwell step, to promote the escape of the injected air and of the removed particles.

In particular, for each nozzle, with regard to the washing of a container 2, i.e. the step during which the nozzle injects the washing fluid into the container 2, the corresponding washing angle β is determined by the position of the nozzle relative to the frame 3 between the initial and final instants of the injection of the fluid. Therefore, the duration of the washing step is proportional to the wash angle β and inversely proportional to the velocity of rotation of the carrousel 4 relative to the frame 3. Similarly, the duration of each of said steps is proportional to the angle corresponding to that step and inversely proportional to the velocity of rotation of the carrousel 4 relative to the frame 3.

The apparatus 1 also comprises a plurality of valves 7 associated with the carrousel 4 and operatively connected to corresponding nozzles to enable them / disable them. Each valve 7 is provided with a control element 8, movable between a first and a second operative position, corresponding to stable opened / closed positions of the valve 7.

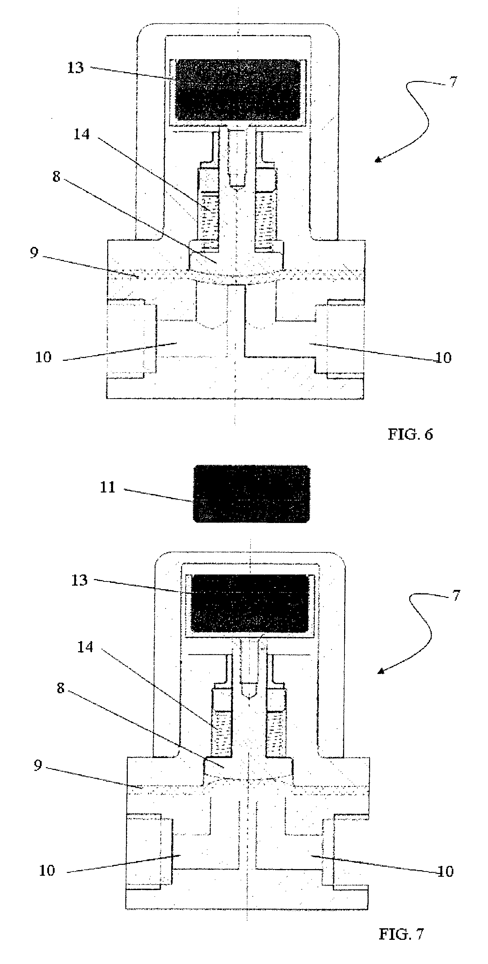

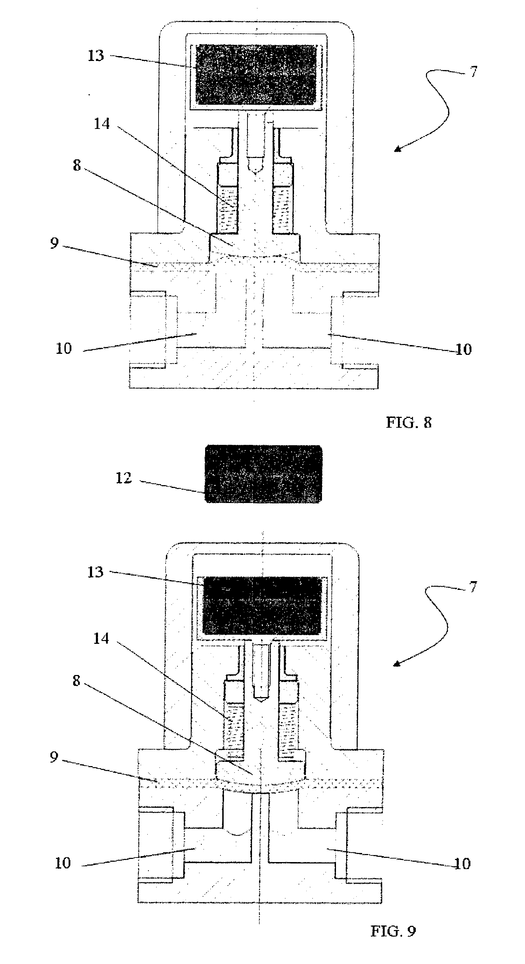

In the illustrated embodiment (in particular in figures 6 - 9), the control element 8 is constituted by a thruster that acts on a membrane 9 housed internally to the valve and able to open and close a conduit 10 for feeding the washing fluid to a nozzle, as a function of the action of the thruster 8 on the membrane 9 itself (according to a substantially known technique). In particular, when the control element 8 is in the first operative open position on the membrane 9 the thruster does not act, this allowing the passage of wash liquid in the conduit 10; when the control element 8 is in the second operative closed position, the membrane 9 is deformed, in such a way as to close / shut off the conduit 10, preventing the passage of wash liquid in the conduit 10 itself.

It should be noted that, in the apparatus 1, to each nozzle corresponds a gripping means and a valve 7, integral with the carrousel 4 and co-operating with each other, in order to perform, for each rotation of the carrousel 4, a treatment on a single corresponding container 2.

Moreover, the apparatus 1 comprises means for activating / deactivating the valves 7, the activation / deactivation of each valve 7 defining said initial and final instants of the injection of the wash fluid into the container 2 corresponding to the valve 7 itself.

Said means for activating / deactivating the valves 7 originally comprise:
- an opening magnet 11, associated with the frame 3 and able to interact magnetically with the control element 8 of a valve 7, when it is in proximity thereto, to bring it to the open position;
- a closing magnet 12, associated with the frame 3 and able to interact magnetically with the control element 8 of a valve 7, when it is in proximity thereto, to bring it to the closed position, said closing magnet 12 being positioned at a predetermined angular distance from the opening magnet 11.

Said opening and closing magnets consist of magnetic dipoles obtained generically, e.g. by means of permanent magnets, or electromagnets, or electro-permanent magnets.

The apparatus 1 also comprises regulating means (not shown because they are substantially known in themselves) connected to a sensor able to detect the presence of a container 2 at a valve 7 and operatively active on the opening magnet 11, to disable it in the absence of containers 2 associated with the pincers, for example moving it radially in a position in which it is not able interact magnetically with the valves 7, thereby allowing to inject the fluid only in the presence of the container in the respective pincer.

In the preferred embodiment illustrated herein, the control element 8 of a valve 7 comprises a thruster provided with permanent magnet 13, able to interact magnetically with the opening magnet 11 or with the closing magnet 12, when the valve 7, rotating integrally with the carrousel 4, is in proximity to said opening or closing magnet.

It should be noted that, in the illustrated embodiment, the valve 7 also comprises a spring 14, or other equivalent elastic means, operatively connected to the thruster 8 to thrust it towards the second operative position, in which said thruster acts on the membrane 9, deforming it and thereby interrupting the conduit 10.

When the valve 7 is in the active position, the washing liquid, flowing through the conduit 10, exercises a pressure on the whole surface of the membrane 9 and hence on the thruster 8, such as to keep it in the first operative position, overcoming the action of the spring 14. When the valve 7 is in the inactive position, the washing liquid, because the conduit 10 is shut off, exercises a pressure on a reduced surface of the membrane 9, so the force that acts on the thruster 8 is relatively modest; in particular, said force is insufficient to overcome the action of the spring 14, therefore leaving the thruster 8 in the second operative position.

Thus, the valve 7 is bistable because it tends to remain in the active position or in the inactive position, in the absence of an intervention of forces from outside the valve itself.

It should be noted that in the washing apparatus 1 according to the present invention any type of bistable valve can be applied, not necessarily of the type comprising a membrane as described above.

When a valve 7 is in proximity to the opening magnet 11, a magnetic force is established between the opening magnet 11 and the control element 8, so that the control element 8 is actuated towards the first operative position, whereto corresponds the active position of the valve 7.

In the illustrated embodiment, said magnetic force is established between the opening magnet 11 and the permanent magnet 13 comprised in the control element 8 (or associated to the control element 8). Preferably, if the control element is constituted by a thruster, the permanent magnet is integrated in a portion of the thruster itself.

When a valve 7 is in proximity to the closing magnet 12, a magnetic force is established between the closing magnet 12 and the control element 8, so that the control element 8 is actuated towards the second operative position, whereto corresponds the inactive position of the valve 7.

In the illustrated embodiment, said magnetic force is established between the closing magnet 12 and the permanent magnet 13 assigned to the control element 8; for this purpose, the closing magnet 12 presents inverted polarity with respect to the opening magnet 11; alternatively, the opening and closing magnets are positioned on the frame 3 in such a way as to be in opposite positions from the permanent magnet 13 of the valve 7, when they are in proximity to the valve.

It should be noted that an embodiment (not shown) is provided, in which the control element 8 comprises a block of ferromagnetic material (instead of the permanent magnet 13), able to interact magnetically with the opening magnet 11 and with the closing magnet 12.

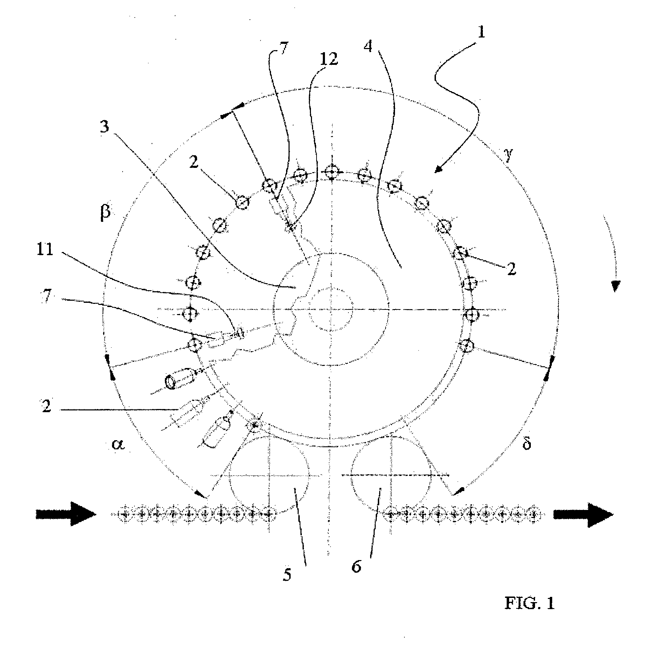

With reference to figure 1, it should be noted that the opening magnet 11 and the closing magnet 12 are positioned on the frame 3 at a predetermined angular distance, which defines the wash angle β, i.e. it defines the washing time for a given velocity of rotation of the carrousel 4.

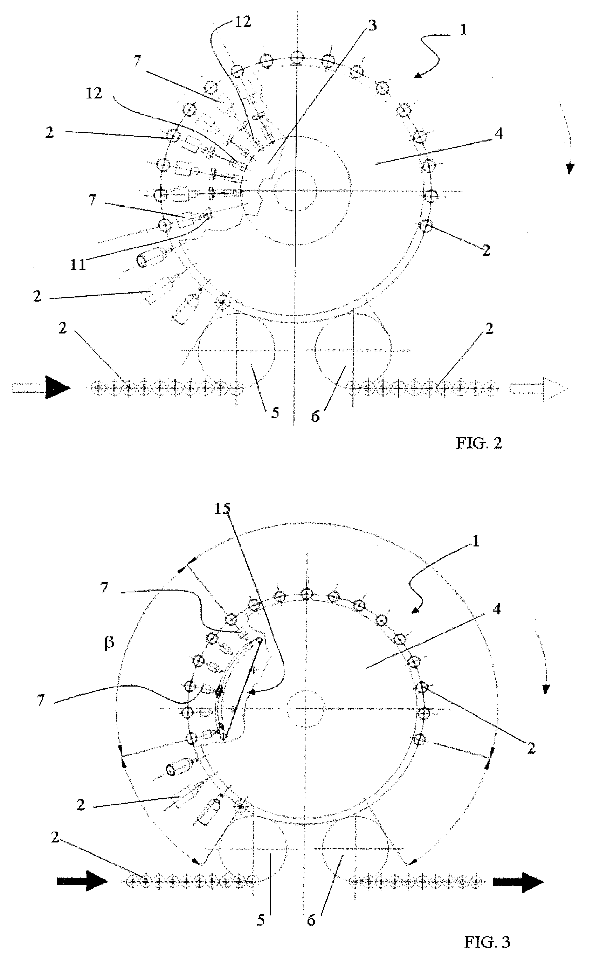

The present invention comprises an embodiment (shown in figure 2), according to which the apparatus 1 originally comprises a plurality of closing magnets 12, each being movable relative to the frame 3 between an active position, in which it is able to interact magnetically with said control element 8 of a valve 7, and a passive position, in which it does not interfere magnetically with said control element 8 of a valve 7. For example, each closing magnet 12 is fastened on a piston that is movable relative to its own cylinder fastened to the frame 3.

In this way, the washing time is discretely variable according to a placement of the closing magnets 12 in active or passive position. The wash angle β is defined by the angular distance between the opening magnet and the first (i.e. the closest) of the closing magnets 12 placed in active position.

Therefore, the present invention advantageously enables to adjust the wash angle β, i.e. the washing time. In particular, the present invention enables to activate / deactivate the closing magnets 12 in such a way that between the instant in which a valve 7 interacts with the opening magnet 11 and the instant in which the same valve interacts with the closing magnet 12 a predetermined washing time elapses, during which said valve is active, as a function of a direction of rotation of the carrousel 4 relative to the frame 3.

It should be noted that the capability of varying the wash angle β can also be obtained through a plurality of opening magnets 11, each being movable relative to the frame 3 between an active position, in which it is able to interact magnetically with said control element 8 of a valve 7, and a passive position, in which it does not interfere magnetically with said control element of a valve, so that the washing time is variable as a function of a placement of the opening magnets 11 in active or passive position. For example, each opening magnet 11 is fastened on a piston that is movable relative to its own cylinder fastened to the frame 3.

The present invention provides for an additional embodiment (shown in figures 3-5 and 10), according to which the apparatus 1 originally comprises actuating means 15, operatively active on the closing magnet 12 and/or on the opening magnet 11, to vary said predetermined distance between the opening magnet 11 and the closing magnet 12.

With regard to the actuating means 15, in particular, three embodiments are provided.

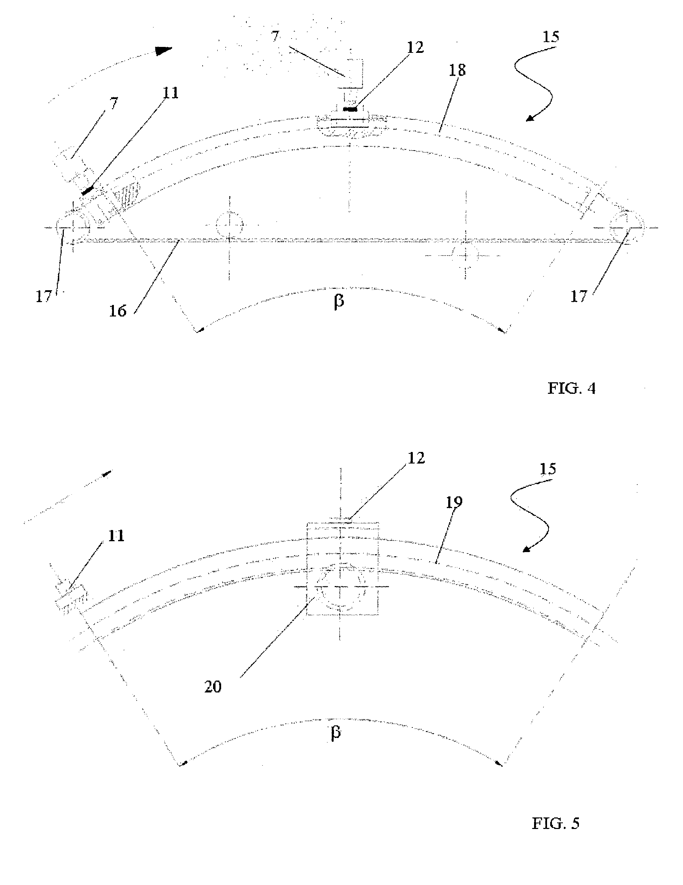

According to a first embodiment (shown in figures 3 and 4), the actuating means 15 comprise a belt 16 (or any other flexible transmission member) connected to pulleys 17 associated to the frame 3, the opening magnet 11 or the closing magnet 12 being anchored to the belt 16. In particular, in the embodiment shown in figures 3 and 4 the closing magnet 12 is anchored to the belt 16, whilst the opening magnet 11 is fixed. It should be noted that a portion of the belt 16 is slidably coupled to a linear guide 18, in such a way as to be positioned along an arc of circumference, which corresponds to a portion of the trajectory of the valves 7 (rotating integrally with the carrousel 4).

In accordance with a second embodiment (shown in figure 5), the actuating means 15 comprise:
- a guide 19 associated to the frame 3 and provided with a toothing;
- a cursor 20 provided with a gear wheel, preferably motorised, meshing in said toothing.

As shown in figure 5, the closing magnet 12 is anchored to the cursor 20, whilst the opening magnet 11 is fixed. Alternatively, the opening magnet 11 is anchored to the cursor 20 and the closing magnet is fixed.

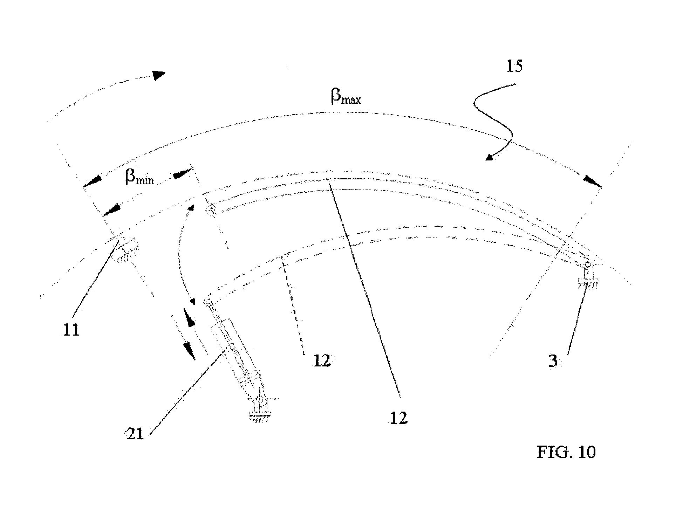

According to a third embodiment (shown in figure 10), the actuating means 15 comprise a cylinder 21 or any other movable element relative to the frame 3 in substantially radial direction. The closing magnet 12 (or the opening magnet 11) preferably has an arched shape, substantially parallel to the development of the circumference defined by the trajectory described by the valves 7 in their rotation. Said magnet presents a first end hinged to the frame 3 and a second end fastened to said cylinder 21. Moving the cylinder 21, the magnet is moved relative to the trajectory of the valve 7; consequently, the point is moved in which the distance between a valve 7 and the magnet is such as to activate said magnetic interaction. In this way, it is possible to vary the wash angle β from a minimum value βₘᵢₙ to a maximum value βₘₐₓ.

It should be noted that the actuating means 15 advantageously enable to vary the wash angle β with continuity and automatically.

In particular, the apparatus 1 comprises an element (not shown because it is known in itself) for measuring the angular velocity of rotation of the carrousel 4 relative to the frame 3, and control means operatively connected to the measuring element and to the actuating means 15 to vary said predetermined distance between the opening magnet 11 and the closing magnet 12 (i.e. to vary the wash angle β), as a function of the measured angular velocity.

In this way, the apparatus 1 advantageously enables to vary automatically, during the operation of the apparatus itself, the wash angle β, in order to maintain substantially constant the washing time as the angular velocity of the carrousel changes; this enables, advantageously, to adapt the treatment angle to the hourly production and to the format of the containers, avoiding longer washing time than necessary, or needless waste of the washing fluid.

It should also be noted that the apparatus 1 according to the present invention exploits, for the activation / deactivation of the valves 7, solely magnetic forces, with the advantage of avoiding mechanical parts (which entail rubbing phenomena, undesired because they are penalising for the reliability and hygiene of the apparatus) and electric / electronic circuitry (which would entail a complication of the apparatus and a consequent reduction in the reliability of the apparatus itself).

## Claims

1. An apparatus (1) for washing/sterilising/blowing containers (2), comprising:
- a frame (3);
- a carrousel (4), rotating relative to said frame (3);
- a plurality of nozzles associated with the carrousel (4), fed with a pressurized washing fluid with a predetermined pressure and co-operating with means for gripping the containers (2) to inject the fluid into the containers (2);
- a plurality of valves (7) associated with the carrousel (4) and operatively connected to corresponding nozzles to enable them / disable them, each valve (7) being provided with a control element (8) movable between a first and a second operative position, corresponding to stable open / closed positions of the valve (7);
- means for activating / deactivating the valves (7),
wherein
the means for activating / deactivating the valves comprise, in combination:
- an opening magnet (11), associated with the frame (3) and able to interact magnetically with a magnet provided on said control element (8) of a valve (7), when it is in proximity thereto, to bring it to the opened position;
- a closing magnet (12), associated with the frame (3) and able to interact magnetically with a magnet provided on said control element (8) of a valve (7), when it is in proximity thereto, to bring it to the closed position, said closing magnet (12) being positioned at a predetermined distance from the opening magnet (11),
said control element (8) being constituted by a thruster that acts on a membrane (9) housed internally to the valve and able to open and close a conduit (10) for feeding the washing fluid to a nozzle, as a function of the action of the thruster (8) on the membrane (9) itself, so that when the control element (8) is in the first operative open position the thruster (8) does not act on the membrane (9), this allowing the passage of washing fluid in the conduit (10) and when the control element (8) is in the second operative closed position, the membrane (9) is deformed, in such a way as to close / shut off the conduit (10), preventing the passage of washing fluid in the conduit (10),
and wherein said membrane (9) is forming the closing wall of a side of the valve seat and can be moved in direction of an opposite wall of the valve seat in which are provided the openings of the two branches of said conduit (10), from the first operative open position in which the membrane (9) is at a predetermined distance away from the two openings to the second operative closed position, in which the membrane (9) lays against said openings, thus closing said openings, **characterised in**
**that**, when the valve (7) is in the first operative open position, the washing fluid, flowing through the conduit (10), exercises said predetermined pressure on the whole surface of the membrane (9) and hence on the thruster (8), the whole surface of the membrane having such an area that the pressure generates a force that keeps it in the first operative open position, said force being sufficient to overcome the action of a spring (14) which thrusts the thruster towards the second operative closed position, and when the valve (7) is in the second operative closed position, the washing fluid exercises the pressure on a reduced surface of the membrane (9), so the force that acts on the thruster (8) is insufficient to overcome the action of the spring (14), therefore the action of the spring (14) leaves the thruster (8) in the second operative closed position, being said valve (7) of bistable type.

2. An apparatus as claimed in claim 1, wherein said control element (8) of a valve (7) comprises a permanent magnet (13) and/or electromagnet.

3. An apparatus as claimed in claim 1, wherein the control element (8) of a valve (7) has at least one portion made of ferromagnetic material.

4. An apparatus as claimed in claim 1, comprising a plurality of closing magnets (12), each being movable relative to the frame (3) between an active position, in which it is able to interact magnetically with said control element (8) of a valve (7), and a passive position, in which it does not interfere magnetically with said control element (8) of a valve (7), so that the time for washing a container (2) is variable as a function of a given placement of the closing magnets (12) in active or passive position.

5. An apparatus as claimed in claim 1, comprising a plurality of opening magnets (11), each being movable relative to the frame (3) between an active position, in which it is able to interact magnetically with said control element (8) of a valve (7), and a passive position, in which it does not interfere magnetically with said control element (8) of a valve (7), so that the time for washing a container (2) is variable as a function of a given placement of the opening magnets (11) in active or passive position.

6. An apparatus as claimed in claim 1, comprising actuating means (15), operatively active on the closing magnet (12) and/or on the opening magnet (11), to vary said predetermined distance between the opening magnet (11) and the closing magnet (12).

7. An apparatus as claimed in claim 6, wherein the actuating means (15) comprise a belt (16) connected to pulleys (17) associated with the frame (3), the opening magnet (11) or the closing magnet (12) being anchored to the belt (16).

8. An apparatus as claimed in claim 6, wherein the actuating means (15) comprise:
- a guide (19) associated to the frame (3) and provided with a toothing;
- a cursor (20) provided with a gear wheel meshing in said toothing,the opening magnet (11) or the closing means (12) being anchored to the cursor (20).

9. An apparatus as claimed in claim 6, comprising:
- an element for measuring the angular velocity of rotation of the carrousel (4) relative to the frame (3);
- control means operatively connected to the measuring element and to the actuating means (15) to vary said predetermined distance between the opening magnet (11) and the closing magnet (12), as a function of the measured angular velocity.

10. An apparatus as claimed in claim 6, wherein the closing magnet (12) and/or the opening magnet (11) has a first end hinged in the frame (3) and a second end connected to said actuating means.

11. An apparatus as claimed in claim 10, wherein said actuating means comprise a cylinder, movable relative to the frame (3) in substantially radial direction, said cylinder being connected to said second end of the magnet.

12. An apparatus as claimed in any of the previous claims, wherein said opening magnet (11) and/or said closing magnet (12) are permanent magnets.

13. An apparatus as claimed in any of the previous claims from 1 through 11, wherein said opening magnet (11) and/or said closing magnet (12) are electromagnets.

## Patentansprüche

1. Vorrichtung (1) zum Waschen/Sterilisieren/Blasen von Behältern (2), mit:
- einem Rahmen (3);
- einem sich relativ zu dem Rahmen (3) drehenden Karussell (4);
- mehreren mit dem Karussell (4) verbundenen Düsen, die mit druckbeaufschlagtem Waschfluid mit vorbestimmtem Druck beschickt werden und mit Vorrichtungen zum Greifen der Behälter (2) zusammenarbeiten, um das Fluid in die Behälter (2) zu injizieren;
- mehreren Ventilen (7), die mit dem Karussell (4) verbunden und betriebsmäßig an entsprechende Düsen angeschlossen sind, um diese zu aktivieren / zu deaktivieren, wobei jedes Ventil (7) mit einem Steuerelement (8) versehen ist, das zwischen einer ersten und einer zweiten Betriebsposition bewegbar ist, die stabilen offenen/geschlossenen Positionen des Ventils (7) entsprechen;
- Vorrichtungen zum Aktivieren/Deaktivieren der Ventile (7),
wobei
die Vorrichtungen zum Aktivieren/Deaktivieren der Ventile in Kombination aufweisen:
- einen Öffnungs-Magneten (11), der mit dem Rahmen (3) verbunden und in der Lage ist, magnetisch mit einem am dem Steuerelement (8) eines Ventils (7) vorgesehenen Magneten zu interagieren, wenn er sich in dessen Nähe befindet, um es in die geöffnete Position zu bringen;
- einen Schließ-Magneten (12), der mit dem Rahmen (3) verbunden und in der Lage ist, magnetisch mit einem am dem Steuerelement (8) eines Ventils (7) vorgesehenen Magneten zu interagieren, wenn er sich in dessen Nähe befindet, um das Ventil in die geschlossene Position zu bringen, wobei der Schließ-Magnet (12) in einem vorbestimmten Abstand von dem Öffnungs-Magneten (11) positioniert ist,
wobei das Steuerelement (8) durch ein Druckteil gebildet ist, das auf eine in dem Ventil untergebrachte Membran (9) einwirkt und in der Lage ist, als Funktion der Einwirkung des Druckteils (8) auf die Membran (9) selbst eine Leitung (10) zum Zuführen des Waschfluids zu einer Düse zu öffnen und zu schließen, so dass, wenn sich das Steuerelement (8) in der ersten, offenen Betriebsposition befindet, das Druckteil (8) nicht auf die Membran (9) einwirkt, wobei dies den Durchtritt von Waschfluid in der Leitung (10) ermöglicht, und, wenn sich das Steuerelement (8) in der zweiten, geschlossenen Betriebsposition befindet, die Membran (9) derart verformt wird, dass die Leitung (10) geschlossen/blockiert wird und somit der Durchtritt von Waschfluid in der Leitung (10) verhindert wird,
und wobei die Membran (9) die Schließwand einer Seite des Ventilsitzes bildet und in Richtung einer gegenüberliegenden Wand des Ventilsitzes, in der die Öffnungen der beiden Abzweige der Leitung (10) angeordnet sind, aus der ersten, offenen Betriebsposition, in der sich die Membran (9) in einem vorbestimmten Abstand von den beiden Öffnungen befindet, in die zweite, geschlossene Betriebsposition bewegt werden kann, in der die Membran (9) gegen die Öffnungen anliegt, so dass die Öffnungen geschlossen sind, **dadurch gekennzeichnet,**
**dass**, wenn sich das Ventil (7) in der ersten, offenen Betriebsposition befindet, das durch die Leitung (10) strömende Waschfluid den vorbestimmten Druck auf die gesamte Oberfläche der Membran (9) und somit auf das Druckteil (8) ausübt, wobei die gesamte Oberfläche der Membran einen derartigen Flächenbereich aufweist, dass der Druck eine Kraft erzeugt, der sie in der ersten, offenen Betriebsposition hält, wobei die Kraft ausreichend ist, um die Wirkung einer Feder (14) zu überwinden, die das Druckteil zu der zweiten, geschlossenen Betriebsposition hin drückt, und, wenn sich das Ventil (7) in der zweiten, geschlossenen Betriebsposition befindet, das Waschfluid den Druck auf eine reduzierte Oberfläche der Membran (9) ausübt, so dass die auf das Druckteil (8) einwirkende Kraft unzureichend ist, um die Wirkung der Feder (14) zu überwinden, und somit die Wirkung der Feder (14) das Druckteil (8) in der zweiten, geschlossenen Betriebsposition belässt, wobei das Ventil (7) vom bistabilen Typ ist.

2. Vorrichtung nach Anspruch 1, bei der das Steuerelement (8) eines Ventils (7) einen Permanentmagneten (13) und/oder Elektromagneten aufweist.

3. Vorrichtung nach Anspruch 1, bei der das Steuerelement (8) eines Ventils (7) mindestens einen Abschnitt aufweist, der aus ferromagnetischem Material ausgebildet ist.

4. Vorrichtung nach Anspruch 1, mit mehreren Schließ-Magneten (12), die jeweils relativ zu dem Rahmen (3) zwischen einer aktiven Position, in der er in der Lage ist, magnetisch mit dem Steuerelement (8) eines Ventils (7) zu interagieren, und einer passiven Position bewegbar sind, in der er nicht magnetisch mit dem Steuerelement (8) eines Ventils (7) interagiert, so dass die Zeit zum Waschen eines Behälters (2) als Funktion einer gegebenen Platzierung der Schließ-Magneten (12) in der aktiven oder passiven Funktion variabel ist.

5. Vorrichtung nach Anspruch 1, mit mehreren Öffnungs-Magneten (11), die jeweils relativ zu dem Rahmen (3) zwischen einer aktiven Position, in der er in der Lage ist, magnetisch mit dem Steuerelement (8) eines Ventils (7) zu interagieren, und einer passiven Position bewegbar sind, in der er nicht magnetisch mit dem Steuerelement (8) eines Ventils (7) interagiert, so dass die Zeit zum Waschen eines Behälters (2) als Funktion einer gegebenen Platzierung der Öffnungs-Magneten (11) in der aktiven oder passiven Funktion variabel ist.

6. Vorrichtung nach Anspruch 1, mit Betätigungsvorrichtungen (15), die betriebsmäßig auf den Schließ-Magneten (12) und/oder auf den Öffnungs-Magneten (11) einwirken, um den vorbestimmten Abstand zwischen dem Öffnungs-Magneten (11) und dem Schließ-Magneten (12) zu variieren.

7. Vorrichtung nach Anspruch 6, bei der die Betätigungsvorrichtungen (15) einen Riemen (16) aufweisen, der mit an dem Rahmen (3) angebrachten Riemenscheiben (17) verbunden ist, wobei der ÖffnungsMagnet (11) oder der Schließ-Magnet (12) an dem Riemen (16) verankert ist.

8. Vorrichtung nach Anspruch 6, bei der die Betätigungsvorrichtungen (15) aufweisen:
- eine Führung (19), die mit dem Rahmen (3) verbunden und mit einer Verzahnung versehen ist;
- einen Kursor (20), der mit einem Zahnrad versehen ist, das mit der Verzahnung kämmt, wobei der Öffnungs-Magnet (11) oder die Schließ-Vorrichtung (12) an dem Kursor (20) verankert ist.

9. Vorrichtung nach Anspruch 6, mit:
- einem Element zum Messen der Winkelgeschwindigkeit der Drehung des Karussells (4) relativ zu dem Rahmen (3);
- betriebsmäßig mit dem Mess-Element und mit den Betätigungsvorrichtungen (15) verbundenen Steuervorrichtungen zum Variieren des vorbestimmten Abstands zwischen dem Öffnungs-Magneten (11) und dem Schließ-Magneten (12) als Funktion der gemessenen Winkelgeschwindigkeit.

10. Vorrichtung nach Anspruch 6, bei der der Schließ-Magnet (12) und/ oder der Öffnungs-Magnet (11) ein erstes Ende, das in dem Rahmen (3) angelenkt ist, und ein zweites Ende hat, das mit den Betätigungsvorrichtungen verbunden ist.

11. Vorrichtung nach Anspruch 10, bei der die Betätigungsvorrichtungen einen Zylinder aufweisen, der relativ zu dem Rahmen (3) im Wesentlichen in radialer Richtung bewegbar ist, wobei der Zylinder mit dem zweiten Ende des Magneten verbunden ist.

12. Vorrichtung nach einem der vorherigen Ansprüche, bei der der Öffnungs-Magnet (11) und/oder der Schließ-Magnet (12) Permanentmagneten sind.

13. Vorrichtung nach einem der vorherigen Ansprüche 1 bis 11, bei der der Öffnungs-Magnet (11) und/oder der Schließ-Magnet (12) Elektromagneten sind.

## Revendications

1. Appareil (1) pour laver/stériliser/souffler des récipients (2), comprenant :
- un bâti (3) ;
- un carrousel (4), tournant par rapport audit bâti (3) ;
- plusieurs buses associées au carrousel (4), alimentées au moyen d'un fluide de lavage sous pression présentant une pression préfixée et coopérant avec des moyens permettant de maintenir les récipients (2) de façon à injecter le fluide dans les récipients (2) ;
- plusieurs soupapes (7) associées au carrousel (4) et reliées de manière opérationnelle à des buses correspondantes de façon à les mettre en service/les mettre hors service, chaque soupape (7) étant pourvue d'un élément de commande (8) mobile entre une première et une seconde positions opérationnelles, correspondant à des positions ouverte/fermée stables de la soupape (7) ;
- des moyens pour activer/désactiver les soupapes (7),
dans lequel
les moyens pour activer/désactiver les soupapes comprennent, en combinaison :
- un aimant d' ouverture (11), associé au bâti (3) et capable de coopérer magnétiquement de manière interactive avec un aimant prévu sur ledit élément de commande (8) d'une soupape (7), lorsqu'il est à proximité de celle-ci, de façon à l'amener à la position ouverte ;
- un aimant de fermeture (12), associé au bâti (3) et capable de coopérer magnétiquement de manière interactive avec un aimant prévu sur ledit élément de commande (8) d'une soupape (7), lorsqu'il est à proximité de celle-ci, de façon à l'amener à la position fermée, ledit aimant de fermeture (12) étant positionné à une distance préfixée à partir de l'aimant d'ouverture (11),
ledit élément de commande (8) étant constitué par un poussoir qui agit sur une membrane (9) logée intérieurement à la soupape et capable d'ouvrir et de fermer un conduit (10) permettant d'acheminer le fluide de lavage à une buse, en fonction de l'action du poussoir (8) sur la membrane (9) elle-même, de sorte que, lorsque l'élément de commande (8) est dans la première position ouverte opérationnelle, le poussoir (8) n'agit pas sur la membrane (9), ce qui permet le passage de fluide de lavage dans le conduit (10), et, lorsque l'élément de commande (8) est dans la seconde position fermée opérationnelle, la membrane (9) est déformée, de manière à fermer/obturer le conduit (10), ce qui empêche le passage de fluide de lavage dans le conduit (10),
et dans lequel ladite membrane (9) forme la paroi de fermeture d'une face du siège de soupape et peut être déplacée en direction d'une paroi opposée du siège de soupape dans laquelle sont prévues les ouvertures des deux branches dudit conduit (10), de la première position ouverte opérationnelle, dans laquelle la membrane (9) est éloignée à une distance préfixée des deux ouvertures, à la seconde position fermée opérationnelle, dans laquelle la membrane (9) repose en appui sur lesdites ouvertures, fermant ainsi lesdites ouvertures,
**caractérisé**
**en ce que**, lorsque la soupape (7) est dans la première position ouverte opérationnelle, le fluide de lavage, s'écoulant par le conduit (10), exerce ladite pression préfixée sur la surface totale de la membrane (9) et par conséquent sur le poussoir (8), la surface totale de la membrane ayant une aire telle que la pression produit une force qui la maintient dans la première position ouverte opérationnelle, ladite force étant suffisante pour surmonter l'action d'un ressort (14) qui repousse le poussoir vers la seconde position fermée opérationnelle, et, lorsque la soupape (7) est dans la seconde position fermée opérationnelle, le fluide de lavage exerce la pression sur une surface réduite de la membrane (9), de sorte que la force qui agit sur le poussoir (8) est insuffisante pour surmonter l'action du ressort (14), l'action du ressort (14) laissant par conséquent le poussoir (8) dans la seconde position fermée opérationnelle, ladite soupape (7) étant de type bistable.

2. Appareil tel que revendiqué à la revendication 1, dans lequel ledit élément de commande (8) d'une soupape (7) comprend un aimant permanent (13) et/ou un électroaimant.

3. Appareil tel que revendiqué à la revendication 1, dans lequel l'élément de commande (8) d'une soupape (7) présente au moins une partie faite de matière ferromagnétique.

4. Appareil tel que revendiqué à la revendication 1, comprenant plusieurs aimants de fermeture (12), chacun étant mobile vis-à-vis du bâti (3) entre une position active, dans laquelle il est capable de coopérer magnétiquement de manière interactive avec ledit élément de commande (8) d'une soupape (7), et une position passive, dans laquelle il n'interfère pas magnétiquement avec ledit élément de commande (8) d'une soupape (7), de sorte que le temps prévu pour le lavage d'un récipient (2) est variable en fonction d'un positionnement donné des aimants de fermeture (12) en position active ou passive.

5. Appareil tel que revendiqué à la revendication 1, comprenant plusieurs aimants d'ouverture (11), chacun étant mobile vis-à-vis du bâti (3) entre une position active, dans laquelle il est capable de coopérer magnétiquement de manière interactive avec ledit élément de commande (8) d'une soupape (7), et une position passive, dans laquelle il n'interfère pas magnétiquement avec ledit élément de commande (8) d'une soupape (7), de sorte que le temps prévu pour le lavage d'un récipient (2) est variable en fonction d'un positionnement donné des aimants d'ouverture (11) en position active ou passive.

6. Appareil tel que revendiqué à la revendication 1, comprenant des moyens d'actionnement (15), agissant d'une manière opérationnelle sur l'aimant de fermeture (12) et/ou sur l'aimant d'ouverture (11), afin de faire varier ladite distance préfixée entre l'aimant d'ouverture (11) et l'aimant de fermeture (12).

7. Appareil tel que revendiqué à la revendication 6, dans lequel les moyens d'actionnement (15) comprennent une courroie (16) reliée à des poulies (17) associées au bâti (3), l'aimant d'ouverture (11) ou l'aimant de fermeture (12) étant fixé à la courroie (16).

8. Appareil tel que revendiqué à la revendication 6, dans lequel les moyens d'actionnement (15) comprennent :
- un guide (19) associé au bâti (3) et pourvu d'une denture;
- un curseur (20) pourvu d'une roue dentée engrenant dans ladite denture, l'aimant d'ouverture (11) ou l'aimant de fermeture (12) étant fixé au curseur (20).

9. Appareil tel que revendiqué à la revendication 6, comprenant :
- un élément permettant de mesurer la vitesse angulaire de rotation du carrousel (4) vis-à-vis du bâti (3) ;
- des moyens de commande reliés de manière opérationnelle à l'élément de mesure et aux moyens d'actionnement (15) de façon à faire varier ladite distance préfixée entre l'aimant d'ouverture (11) et l'aimant de fermeture (12), en fonction de la vitesse angulaire mesurée.

10. Appareil tel que revendiqué à la revendication 6, dans lequel l'aimant de fermeture (12) et/ou l'aimant d'ouverture (11) présente une première extrémité montée de manière articulée dans le bâti (3) et une seconde extrémité reliée auxdits moyens d'actionnement.

11. Appareil tel que revendiqué à la revendication 10, dans lequel lesdits moyens d'actionnement comprennent un vérin, mobile vis-à-vis du bâti (3) dans une direction sensiblement radiale, ledit vérin étant relié à ladite seconde extrémité de l'aimant.

12. Appareil tel que revendiqué dans l'une quelconque des revendications précédentes, dans lequel ledit aimant d'ouverture (11) et/ou ledit aimant de fermeture (12) sont des aimants permanents.

13. Appareil tel que revendiqué dans l'une quelconque des revendications précédentes 1 à 11, dans lequel ledit aimant d'ouverture (11) et/ou ledit aimant de fermeture (12) sont des électroaimants.
